(19) Europäisches Patentamt
European Patent Office
Office européen des brevets

(11) EP 3 816 145 B1

(12) EUROPÄISCHE PATENTSCHRIFT

(45) Veröffentlichungstag und Bekanntmachung des Hinweises auf die Patenterteilung:
**06.07.2022 Patentblatt 2022/27**

(21) Anmeldenummer: **19020610.2**

(22) Anmeldetag: **31.10.2019**

(51) Internationale Patentklassifikation (IPC):
***C07C 29/151*** *(2006.01)* ***C07C 31/04*** *(2006.01)*
***C01B 3/38*** *(2006.01)*

(52) Gemeinsame Patentklassifikation (CPC):
(C-Sets verfügbar)
**C01B 3/382; C07C 29/151; C07C 29/1518;**
C01B 2203/043; C01B 2203/061 (Forts.)

(54) **VERFAHREN UND ANLAGE ZUR HERSTELLUNG VON METHANOL AUS WASSERSTOFFREICHEM SYNTHESEGAS**

METHOD AND INSTALLATION FOR PRODUCING METHANOL FROM HYDROGEN-RICH SYNTHESIS GAS

PROCÉDÉ ET SYSTÈME DE FABRICATION DE MÉTHANOL À PARTIR DE GAZ DE SYNTHÈSE RICHE EN HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.05.2021 Patentblatt 2021/18**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
- **Gronemann, Veronika**
  **61184 Karben (DE)**
- **Haase, Stephan**
  **61449 Steinbach (DE)**
- **Janko, Lutz**
  **64390 Erzhausen (DE)**
- **Kopetsch, Hans**
  **61352 Bad Homburg (DE)**
- **Lim, Chin Han**
  **60438 Frankfurt am Main (DE)**

(74) Vertreter: **Stang, Stefan**
**Air Liquide Forschung und Entwicklung GmbH**
**Gwinnerstraße 27-33**
**60388 Frankfurt am Main (DE)**

(56) Entgegenhaltungen:
**EP-A1- 3 205 622 DE-T5-112006 001 310**

EP 3 816 145 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 29/151, C07C 31/04;**
**C07C 29/1518, C07C 31/04**

## Beschreibung

### Gebiet der Erfindung

Die Erfindung betrifft ein Verfahren sowie eine Anlage zur Herstellung von Methanol aus einem wasserstoffarmen Frischgasstrom, wobei dem wasserstoffarmen Frischgasstrom ein wasserstoffhaltiger Strom zugeführt wird, so dass ein wasserstoffreicher Synthesegasstrom mit einer Stöchiometriezahl von 2.0 oder größer erhalten wird. Die Erfindung betrifft ferner die Verwendung des erfindungsgemäßen Verfahrens oder der erfindungsgemäßen Anlage zur Herstellung von Methanol aus durch autotherme Reformierung und/oder partielle Oxidation erzeugtem Frischgas.

### Stand der Technik

Methanol wird im großtechnischen Maßstab aus Synthesegas hergestellt. Synthesegas ist ein Gemisch aus vorwiegend Wasserstoff ($H_2$), Kohlenmonoxid (CO) sowie Kohlendioxid ($CO_2$). Darüber hinaus weist es kleinere Mengen an unter den Bedingungen der Methanolsynthese inerten Gasbestandteilen auf. Kohlenmonoxid sowie Kohlendioxid werden dabei häufig unter dem Begriff "Kohlenoxide" oder "Kohlenstoffoxide" zusammengefasst. Im heute als Niederdruck-Methanolsynthese bezeichneten Prozess wird das Synthesegas bei einem Synthesedruck von 60 bis 120 bar zu Methanol und Wasser (als zwangsläufig anfallendes Nebenprodukt) umgesetzt. Das eingesetzte Synthesegas, häufig als Frischgas bezeichnet, wird dabei nach Verdichtung auf den jeweiligen Synthesedruck bei Katalysator-Temperaturen von üblicherweise größer als 200 °C durch ein Katalysatorbett eines Methanolsynthese-Katalysators geleitet. Bei dem Methanolsynthese-Katalysator handelt es sich üblicherweise um eine als katalytisch aktive Spezies Kupfer aufweisende Zusammensetzung. Je nach Verfahrensführung kommen eine oder mehrere hintereinander oder parallel geschaltete Reaktoren zum Einsatz, welche jeweils über ein entsprechendes Katalysatorbett verfügen. Dabei ist die Umsetzung der Kohlenstoffoxide zu Methanol und Wasser am Katalysator aufgrund der Einstellung eines thermodynamischen Gleichgewichts gemäß der Reaktionen

$$CO_2 + 3\ H_2 \rightleftharpoons CH_3OH + H_2O,$$

$$CO + 2\ H_2 \rightleftharpoons CH_3OH$$

nicht vollständig. Aus diesem Grund wird der Herstellungsprozess üblicherweise als Kreislaufprozess in einer sogenannten Syntheseschleife (loop) gefahren. Dabei wird das am Reaktorausgang erhaltene Reaktionsgemisch unter den Siedepunkt von Methanol abgekühlt, um Methanol und Wasser aus dem Kreislauf abzutrennen. Gleichzeitig wird nicht umgesetztes Synthesegas zur erneuten Reaktion zum Methanolsynthese-Katalysator zurückgeführt. Vom nicht umgesetzten Synthesegas wird kontinuierlich ein Teilstrom als Spülgasstrom (purge) entnommen, um die Erhöhung der Konzentration von inerten Bestandteilen in der Syntheseschleife mit fortschreitender Zeit zu vermeiden.

Die Zusammensetzung des Frischgases oder eines Synthesegases im Allgemeinen ist durch die sogenannte Stöchiometriezahl SN, definiert als

$$SN = \frac{n(H_2) - n(CO_2)}{n(CO) + n(CO_2)}, mit\ n\ in\ [mol],$$

charakterisiert. Eine für die Methanolsynthese stöchiometrisch ausbilanzierte Frischgaszusammensetzung ist durch eine Stöchiometriezahl SN von 2,0 gekennzeichnet.

Werte von kleiner als 2,0 weisen auf ein Wasserstoffdefizit hin, während Werte von größer als 2,0 auf einen Wasserstoffüberschuss hinweisen.

Synthesegase mit Wasserstoffdefizit werden beispielsweise bei Prozessen, welche einen partiellen Oxidationsschritt umfassen, oder bei der Erzeugung von Synthesegas durch Kohlevergasung, erhalten. In einem solchen Fall wird der Wasserstoff bei der Methanolsynthese nahezu vollständig verbraucht, während ein wesentlicher Teil der Kohlenstoffoxide nicht umgesetzt wird. Dies führt zu einer Zusammensetzung in der Syntheseschleife, welche sich durch hohe Anteile an Kohlenstoffoxiden, jedoch einen niedrigen Anteil an Wasserstoff auszeichnen. Dies hat unter anderem zur Folge, dass der Methanolsynthese-Reaktor mit einem hohen Katalysatorvolumen auszulegen ist und dass der Gehalt an Nebenprodukten (insbesondere höhere Alkohole und Ketone) höher ist als erwünscht.

Um auch wasserstoffarmes Synthesegas sinnvoll großtechnisch bei der Methanolherstellung verwenden zu

können, kann das Synthesegas beispielsweise durch Wasserstoff aus einer Wasserstoffrückgewinnungsanlage auf die gewünschte Stöchiometriezahl von zwei oder größer eingestellt werden. Dies ist beispielsweise durch Wasserstoffrückgewinnung aus dem Spülstrom möglich.

DE 11 2006 001310 T5 beschreibt eine Methanolsynthese aus einem Synthesegas, das einen Wasserstoffmangel aufweist. EP 3 205 622 B1 offenbart ein Verfahren, bei dem als Restgas bezeichnetes nicht umgesetztes Synthesegas teilweise (als Spülgas) einer Wasserstoffrückgewinnungsstufe zugeführt wird. Dabei wird ein wasserstoffhaltiger Strom gewonnen, welcher dem Frischgasstrom zugeführt wird. Das resultierende Gemisch wird anschließend auf Synthesedruck verdichtet und zu Methanol umgesetzt.

Die aus dem Teilstrom des nicht umgesetzten Synthesegases zu gewinnenden Wasserstoffmengen sind jedoch häufig nicht ausreichend, um ein Synthesegas mit ausreichend hoher Stöchiometriezahl zu erhalten. Beispielsweise können Synthesegase mit einem hohen Wasserstoffdefizit einen so hohen Spülstrom-Anteil zur Wasserstoffrückgewinnung erfordern, dass die Syntheseschleife entweder bei niedrigen Drücken betrieben werden muss oder dass das Verhältnis des Rückführgasstroms zum Frischgasstrom niedrig eingestellt werden muss.

Um diesen Nachteilen zu begegnen, ist auch denkbar einen Teil des Frischgases vor der Methanolsynthese abzuzweigen und einer Wasserstoffrückgewinnungsstufe zuzuführen. Der Nachteil dieser Anordnung besteht darin, dass ein Teil des Wasserstoffs in der Wasserstoffrückgewinnungsstufe verloren geht, bevor er in den Synthesekreislauf gelangt. Weiterhin weist das Synthesegas nach der Anreicherung mit diesem Wasserstoff eine Stöchiometriezahl von größer als zwei auf, was dazu führen kann, dass der in diesem Fall nicht genutzte Spülgasstrom zu einem erheblichen Teil nicht umgesetzten Wasserstoff aufweisen kann.

US 7,786,180 B2 schlägt daher vor, der Wasserstoffrückgewinnungsstufe einen gemischten Strom aus Frischgas und Spülgas zuzuführen, um die oben genannten Nachteilen zumindest teilweise zu überwinden. Nachteilig an dieser Anordnung ist beispielsweise, dass der Frischgasstrom durch ein Druckminderungsventil gedrosselt werden muss, um zumindest den durch die Wasserstoffrückgewinnungsstufe erzeugten Druckverlust auszugleichen. Der in der Frischgasleitung dadurch verlorene Druck muss bei der anschließenden Verdichtung auf Synthesedruck kompensiert werden.

**Beschreibung der Erfindung**

Eine Aufgabe der vorliegenden Erfindung besteht darin ein Verfahren und eine Anlage zur Herstellung von Methanol bereitzustellen, welches die Nachteile des Standes der Technik zumindest teilweise überwindet. Insbesondere besteht eine Aufgabe der vorliegenden Erfindung darin ein Verfahren und eine Anlage bereitzustellen, welches keine Drosselung des Hauptfrischgasstroms durch eine Druckminderungsvorrichtung erfordert.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zur Herstellung von Methanol, wobei einem Frischgasstrom aus einer Reformereinheit, welcher Wasserstoff und Kohlenoxide umfasst, ein wasserstoffhaltiger Strom aus einer Wasserstoffrückgewinnungsstufe zugeführt wird, wobei ein wasserstoffreicher Synthesegasstrom mit einer Stöchiometriezahl SN, definiert als $SN = [n(H_2)-n(CO_2)] / [n(CO) + n(CO_2)]$, von 2.0 oder größer erhalten wird, und wobei der wasserstoffreiche Synthesegasstrom mit einem Restgasstrom zusammengeführt und der wasserstoffreiche Synthesegasstrom und der Restgasstrom bei erhöhtem Druck und erhöhter Temperatur durch ein Bett eines Methanolsynthese-Katalysators geleitet werden, wobei ein Produktstrom erhalten wird, welcher Methanol und den Restgasstrom umfasst, und wobei der Produktstrom gekühlt wird um Methanol vom Restgasstrom abzutrennen. Erfindungsgemäß ist vorgesehen, dass von dem Restgasstrom ein Teil als Spülgasstrom abgetrennt wird und von dem wasserstoffreichen Synthesegasstrom ein Teil abgetrennt und mit dem Spülgasstrom zusammengeführt wird, wobei ein gemischter Synthesegasstrom erhalten wird, und der gemischte Synthesegasstrom der Wasserstoffrückgewinnungsstufe zur Erzeugung des wasserstoffhaltigen Stroms zugeführt wird.

Erfindungsgemäß wird nicht der Frischgasstrom und der aus dem Restgasstrom abgezweigte Spülgasstrom der Wasserstoffrückgewinnungsstufe zugeführt, sondern der bereits mit Wasserstoff auf eine Stöchiometriezahl von 2.0 oder größer eingestellte wasserstoffreiche Synthesegasstrom wird zusammen mit dem Spülgasstrom der Wasserstoffrückgewinnungsstufe zugeführt. Dadurch kann auf eine Drosselung des Frischgasstroms zum Abzweigen eines Teils des Frischgasstroms in Richtung der Wasserstoffrückgewinnungsstufe verzichtet werden. Ferner haben Untersuchungen gezeigt, dass durch die erfindungsgemäße Verfahrensführung Einsparungen in Bezug auf die aufzuwendende

Kompressionsenergie erzielt werden können.

Bei dem Frischgasstrom handelt es sich vorzugsweise um einen Synthesegasstrom aus einer Reformereinheit, welcher sich insbesondere durch ein Defizit an Wasserstoff auszeichnet, so dass die Stöchiometriezahl des Frischgases insbesondere bei kleiner als 2.0 liegt. Ein solcher Frischgasstrom wird insbesondere in einer Reformereinheit erzeugt, welche zur Erzeugung des Synthesegases einen partiellen Oxidationsschritt eines kohlenstoffhaltigen Einsatzgases umfasst. Beispielsweise kann der Frischgasstrom aus autothermer Reformierung eines kohlenstoffhaltigen Einsatzgases erzeugt werden. Bei dem Einsatzgas handelt es sich vorzugsweise um Erdgas. Ferner kann der Frischgasstrom aus Kohlevergasung erzeugt werden. Der Frischgasstrom wird vor dem Zuführen des wasserstoffhaltigen Stroms und Verdichtung auf Synthesedruck, zur Kondensation und Abtrennung von Wasser, auf eine Temperatur von vorzugsweise 40 °C oder weniger abgekühlt. Der Frischgasstrom weist üblicherweise einen Druck zwischen 35 und 60 bar auf, weshalb vor der Umsetzung am Methanolsynthese-Katalysator eine zusätzliche Verdichtung auf Synthesedruck erforderlich ist.

Eine Reformereinheit kann eine Einheit zur Umwandlung (Reformierung) eines gasförmigen kohlenstoffhaltigen Einsatzstoffes oder eines festen kohlenstoffhaltigen Einsatzstoffes umfassen. Ein Beispiel für einen gasförmigen kohlenstoffhaltigen Einsatzstoff ist Erdgas. Beispiele für feste kohlenstoffhaltige Einsatzstoffe sind Kohle, feste Abfälle (Müll) und Biomasse.

Der wasserstoffhaltige Strom weist vorzugsweise einen Wasserstoff-Gehalt von 95 Vol.-% oder mehr auf. Angestrebt wird ein wasserstoffhaltiger Strom, welcher reinen oder im Wesentlichen reinen Wasserstoff enthält. Außer dem wasserstoffhaltigen Strom wird durch die Wasserstoffrückgewinnungsstufe auch ein Abgasstrom erzeugt, welcher unter den Bedingungen der Methanolsynthese inerte Bestandteile sowie kleinere Mengen an nicht umgesetzten Kohlenoxiden umfasst.

Am Methanolsynthese-Katalysator erfolgt die Umsetzung des wasserstoffreichen Synthesegasstroms und des Restgasstroms zu Methanol (und Wasser). Die Umsetzung erfolgt in einer Syntheseschleife, das heißt am Katalysator nicht umgesetztes Synthesegas wird als Restgasstrom zum Eingang des betreffenden Reaktors zurückgeführt (recycelt) und am Methanolsynthese-Katalysator zusammen mit erstmalig verwendetem wasserstoffreichem Synthesegas zu Methanol umgesetzt. Die Umsetzung am Methanolsynthese-Katalysator erfolgt vorzugsweise bei einer Katalysatortemperatur von 220 °C bis 270 °C und vorzugsweise einem Druck von 55 bar bis 80 bar. Die Umsetzung am Methanolsynthese-Katalysator findet vorzugsweise in einer oder mehreren hintereinander oder parallel geschalteten Reaktorstufen statt, wobei jede der Reaktorstufen ein entsprechendes Katalysatorbett umfasst. Insbesondere umfassen die Reaktorstufen einen wassergekühlten Reaktor und einen dem wassergekühlten Reaktor nachgeschalteten gasgekühlten Reaktor. Geeignete Katalysatoren sind aus dem Stand der Technik bekannte Materialien auf Kupfer-Basis mit Kupfer als katalytisch aktiver Spezies, ein Beispiel hierfür ist eine Katalysator-Zusammensetzung welche Kupfer, Zinkoxid sowie Aluminiumoxid umfasst.

Eine bevorzugte Ausführungsform des erfindungsgemäßen Verfahrens ist dadurch gekennzeichnet, dass der wasserstoffreiche Synthesegasstrom verdichtet wird, und von dem verdichteten wasserstoffreichen Synthesegasstrom ein Teil abgetrennt und mit dem Spülgasstrom zusammengeführt wird. Vorzugsweise wird der wasserstoffreiche Synthesegasstrom auf Synthesedruck verdichtet. Vorzugsweise wird der wasserstoffreiche Synthesegasstrom auf einen Druck von 70 bar oder mehr, und bis zu einem Druck von 90 bar verdichtet. Wie Untersuchungen gezeigt haben und im Detail weiter unten ausgeführt, werden durch diese Art der Verfahrensführung Einsparungen bei der benötigten Kompressionsenergie erzielt. Vorzugsweise wird in diesem Zusammenhang der Restgasstrom verdichtet und mit dem verdichteten wasserstoffreichen Synthesegasstrom zusammengeführt, und die zusammengeführten Ströme werden durch das Bett des Methanolsynthese-Katalysators geleitet. Der Spülgasstrom wird insbesondere vor der Verdichtung des Restgasstroms von dem Restgasstrom abgezweigt. Vorzugsweise wird der Restgasstrom auf Synthesedruck verdichtet. Vorzugsweise wird der Restgasstrom dabei auf einen Druck von 70 bar oder mehr, und bis zu einem Druck von 90 bar verdichtet.

Eine bevorzugte Ausführungsform des erfindungsgemäßen Verfahrens ist dadurch gekennzeichnet, dass der wasserstoffhaltige Strom durch einen Wasserstoffkompressor verdichtet wird, und der verdichtete wasserstoffhaltige Strom mit dem Frischgasstrom zusammengeführt wird, wobei der wasserstoffreiche Synthesegasstrom erhalten wird, und von dem wasserstoffreichen Synthesegasstrom ein Teil abgetrennt und mit dem Spülgasstrom zusammengeführt wird. Wie Untersuchungen gezeigt haben und weiter unten ausgeführt, werden durch diese Art der Verfahrensführung Einsparungen bei der benötigten Kompressionsenergie erzielt. Der wasserstoffhaltige Strom wird durch den Wasserstoffkompressor auf einen Druck verdichtet, welcher in etwa 1 bis 2 bar oberhalb des Drucks des Frischgases (ca. 35 bis 60 bar) liegt. Vorzugsweise werden in diesem Zusammenhang der wasserstoffreiche Synthesegasstrom und der Restgasstrom gemeinsam verdichtet und durch das Bett des Methanolsynthese-Katalysators geleitet. Vorzugsweise werden der wasserstoffreiche Synthesegasstrom und der Restgasstrom gemeinsam auf Synthesedruck verdichtet. Insbesondere werden der wasserstoffreiche Synthesegasstrom und der Restgasstrom dabei gemeinsam auf einen Druck von 70 bar oder mehr und bis zu einem Druck von 90 bar verdichtet. Der Spülgasstrom wird dabei zwangsläufig vor der gemeinsamen Verdichtung des Restgasstroms und des wasserstoffreichen Synthesegasstroms von dem Restgasstrom abgezweigt.

Eine bevorzugte Ausführungsform des erfindungsgemäßen Verfahrens ist dadurch gekennzeichnet, dass der Stoffmengenstrom-Anteil des wasserstoffreichen Synthesegasstroms am gemischten Synthesegasstrom zwischen 0,10 und 0,95 liegt, bevorzugt zwischen 0,20 und 0,90 liegt, weiter bevorzugt zwischen 0,30 und 0,80 liegt und weiter bevorzugt zwischen 0,50 und 0,75 liegt.

Der Stoffmengenstrom kann beispielsweise durch die Einheit "kmol/h" (kilomol pro Stunde) wiedergegeben werden.

Eine bevorzugte Ausführungsform des erfindungsgemäßen Verfahrens ist dadurch gekennzeichnet, dass der Stoffmengenstrom-Anteil des vom wasserstoffreichen Synthesegasstrom abgetrennten Teils in Bezug auf den gesamten Stoffmengenstrom an wasserstoffreichem Synthesegas zwischen 0,001 und 0,999 liegt, vorzugsweise zwischen 0,005 und 0,800 liegt, weiter bevorzugt zwischen 0,010 und 0,500 liegt, weiter bevorzugt zwischen 0,020 und 0,200 liegt und weiter bevorzugt zwischen 0,050 und 0,100 liegt.

Eine bevorzugte Ausführungsform des erfindungsgemäßen Verfahrens ist dadurch gekennzeichnet, dass der wasserstoffreiche Synthesegasstrom eine Stöchiometriezahl SN von 2,00 bis 2,20 aufweist, bevorzugt von 2,02 bis 2,10 aufweist, und weiter bevorzugt von 2,05 bis 2,07 aufweist.

Eine bevorzugte Ausführungsform des erfindungsgemäßen Verfahrens ist dadurch gekennzeichnet, dass der Frischgasstrom eine Stöchiometriezahl SN von kleiner als 2,0 aufweist, bevorzugt von 1,70 bis 1,95 aufweist, weiter bevorzugt von 1,75 bis 1,90 aufweist, und weiter bevorzugt von 1,78 bis 1,85 aufweist. Synthesegas aus autothermer Reformierung weist häufig eine Stöchiometriezahl um 1,80 auf.

Eine bevorzugte Ausführungsform des erfindungsgemäßen Verfahrens ist dadurch gekennzeichnet, dass die Wasserstoffrückgewinnungsstufe eine Wechseldruckabsorptionsvorrichtung zur Abtrennung von Wasserstoff aus dem gemischten Synthesegasstrom umfasst. Durch eine Wechseldruckabsorptionsvorrichtung kann reiner oder zumindest nahezu reiner Wasserstoff bei hohen Drücken, beispielsweise bei 40 bis 60 bar, erzeugt werden. Wird Wasserstoff bereits bei hohem Druck durch die Wasserstoffrückgewinnungsstufe bereitgestellt, können nachfolgend angeordnete Verdichterstufen, beispielsweise zur Verdichtung von Wasserstoff (Wasserstoffkompressor) oder zur Verdichtung des wasserstoffreichen Synthesegasstroms entsprechend kleiner ausgelegt werden. Ferner steigt die Konzentration an inerten Bestandteilen in der Syntheseschleife umso langsamer an, je höher die Reinheit des in der Wasserstoffrückgewinnungsstufe erzeugten Wasserstoffs ist.

Alternativ zu einer Wechseldruckabsorptionsvorrichtung kann die Wasserstoffrückgewinnungsstufe auch eine Membrantrennstufe zur Abtrennung von Wasserstoff aus dem gemischten Synthesegasstrom umfassen. Ebenso denkbar sind Kombinationen aus einer oder mehreren Wechseldruckabsorptionsvorrichtungen und einer oder mehreren Membrantrennstufen.

Eine bevorzugte Ausführungsform des erfindungsgemäßen Verfahrens ist dadurch gekennzeichnet, dass der wasserstoffhaltige Strom einen Wasserstoffanteil von mindestens 95 Vol.-% aufweist, bevorzugt von mindestens 99 Vol.-% aufweist, weiter bevorzugt von mindestens 99,5 Vol.-% aufweist, weiter bevorzugt von mindestens 99,9 Vol.-% aufweist.

Die vorgenannten Aufgaben werden ferner zumindest teilweise gelöst durch eine Anlage zur Herstellung von Methanol, aufweisend folgende miteinander in Fluidverbindung stehende Anlagenkomponenten: Eine Reformereinheit zur Erzeugung eines Wasserstoff und Kohlenstoffoxide aufweisenden Frischgasstroms; eine Wasserstoffrückgewinnungsstufe zur Erzeugung eines wasserstoffhaltigen Stroms, wobei die Reformereinheit und die Wasserstoffrückgewinnungsstufe so konfiguriert sind, dass aus dem wasserstoffhaltigen Strom und dem Frischgasstrom ein wasserstoffreicher Synthesegasstrom mit einer Stöchiometriezahl SN, definiert als $SN = [n(H_2) - n(CO_2)] / [n(CO) + n(CO_2)]$, von 2.0 oder größer erhältlich ist; eine Reaktorstufe umfassend ein Methanolsynthese-Katalysatorbett, wobei die Reaktorstufe so konfiguriert ist, dass der wasserstoffreiche Synthesegasstrom sowie ein Restgasstrom bei erhöhtem Druck und erhöhter Temperatur durch das Methanolsynthese-Katalysatorbett hindurchgeleitet werden können, wodurch ein Produktstrom erhältlich ist, welcher Methanol und den Restgasstrom umfasst; eine Kühlvorrichtung zum Kühlen des Produktstroms, wobei die Kühlvorrichtung so konfiguriert ist, dass Methanol vom Restgasstrom abgetrennt werden kann. Erfindungsgemäß ist vorgesehen, dass die Anlage so konfiguriert ist, dass von dem Restgasstrom ein Teil als Spülgasstrom abgetrennt werden kann und von dem Synthesegasstrom ein Teil abgetrennt und mit dem Spülgasstrom zusammengeführt werden kann, wodurch ein gemischter Synthesegasstrom erhältlich ist, und der gemischte Synthesegasstrom der Wasserstoffrückgewinnungsstufe zur Erzeugung des wasserstoffhaltigen Stroms zugeführt werden kann.

Die vorgenannten Aufgaben werden ferner zumindest teilweise gelöst durch die Verwendung des erfindungsgemäßen Verfahrens oder der erfindungsgemäßen Anlage zur Herstellung von Methanol aus durch autotherme Reformierung und/oder partielle Oxidation erzeugtem Frischgas.

## Ausführungsbeispiele

Die Erfindung wird im Folgenden durch zwei erfindungsgemäße Beispiele sowie ein Vergleichsbeispiel näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Ausführungsbeispiele in Zusammenhang mit den Zeichnungen und den Zahlenbeispielen.

Es zeigt

Figur 1 ein schematisches Blockfließbild eines Herstellverfahrens oder einer Anlage 100 für die Methanolsynthese gemäß einem ersten Ausführungsbeispiel der Erfindung,

Figur 2 ein schematisches Blockfließbild eines Herstellverfahrens oder einer Anlage 200 für die Methanolsynthese gemäß einem zweiten Ausführungsbeispiel der Erfindung,

Figur 3 ein schematisches Blockfließbild eines Herstellverfahrens oder einer Anlage 300 für die Synthese von Methanol gemäß Stand der Technik.

Bei der Verfahrensführung gemäß Figur 1 wird ein Frischgasstrom 11, beispielsweise erzeugt in einer Anlage zur autothermen Reformierung von Erdgas (nicht gezeigt), mit einem wasserstoffhaltigen Strom 12 zusammengeführt, wodurch ein wasserstoffreicher Synthesegasstrom 13 mit einer Stöchiometriezahl von 2.0 oder größer erzeugt wird. Der wasserstoffreiche Synthesegasstrom 13 wird durch eine Kompressorstufe 30 auf Synthesedruck verdichtet. Von dem wasserstoffreichen Synthesegasstrom 13 wird ein Teil als wasserstoffreicher Synthesegasteilstrom 14 abgetrennt und mit einem Spülgasstrom 15 zusammengeführt, wodurch ein gemischter Synthesegasstrom 16 resultiert. Der gemischte Synthesegasstrom 16 wird der Wasserstoffrückgewinnungsstufe 31 zugeführt, in welcher durch Wechseldruckabsorption der wasserstoffhaltige Strom 12 mit einem Wasserstoff-Anteil von mindestens 99 Vol.-% erzeugt wird. Gleichzeitig in der Wasserstoffrückgewinnungsstufe 31 erzeugtes Abgas 17, welches Kohlenstoffoxide sowie unter den Bedingungen der Methanolsynthese inerte Bestandteile enthält, kann beispielsweise als Brenngas in der der Methanolsynthese vorgeschalteten Reformereinheit verwendet werden.

Der Hauptteil 18 des auf Synthesedruck verdichteten wasserstoffreichen Synthesegasstroms wird mit in einer Kompressorstufe 32 auf Synthesedruck verdichteten Restgasstrom 19 zusammengeführt. Der resultierende kombinierte Synthesegasstrom 20 wird in einem Wärmeaustauscher 33 aufgeheizt und als aufgeheizter Synthesegasstrom 21 einem Methanol-Reaktor 34 zugeführt. Im Methanol-Reaktor 34 findet die Umsetzung des Synthesegases aus Synthesegasstrom 21 am Methanolsynthese-Katalysator des Katalysatorbetts 35 zu Methanol und Wasser statt. Der aus der Umsetzung im Reaktor 34 resultierende Produktstrom 22, welcher neben Methanol und Wasser auch nicht reagiertes Synthesegas oder Restgas umfasst, wird anschließend nacheinander über die Wärmeaustauscher 36, 33 und 37 gekühlt, wobei den jeweiligen Wärmeaustauschern nachgeschaltet jeweils die Produktströme 23, 24 und 25 resultieren. Anschließend erfolgt in einem Abscheider 38 die Trennung des gekühlten Produktstroms 25 in eine Methanol und Wasser aufweisende flüssige Phase sowie eine Restgas umfassende gasförmige Phase. Aus dem Abscheider 38 wird das nicht im Reaktor 34 umgesetzte Synthesegas oder Restgas als Restgasstrom 26 abgezogen. Gleichzeitig wird ein Methanol und Wasser aufweisender Rohmethanolstrom 27 aus dem Abscheider 38 abgezogen und der weiteren Aufarbeitung, beispielsweise einer Rektifikation (nicht gezeigt), zugeführt. Von dem Restgasstrom 26 wird der Spülgasstrom 15 abgetrennt und ein verbleibender Restgasstrom 28 in der Kompressorstufe 32 auf Synthesedruck verdichtet. Auf Synthesedruck verdichteter Restgasstrom 19 wird wiederum mit wasserstoffreichem Synthesegasstrom 18 zusammengeführt und erneut der Umsetzung zu Methanol im Methanol-Reaktor 34 zugeführt.

Figur 2 zeigt eine gegenüber dem Beispiel der Figur 1 abgewandelte Art der Verfahrensführung gemäß einem weiteren Beispiel der Erfindung. Nach der Verfahrensführung gemäß Figur 2 wird der in der Wasserstoffrückgewinnungsstufe 31 erzeugte wasserstoffhaltige Strom 12 in einem Wasserstoffkompressor 40 verdichtet, wobei ein verdichteter wasserstoffhaltiger Strom 51 erhalten wird, der mit dem Frischgasstrom 11 zusammengeführt wird. Dabei resultiert ein wasserstoffreicher Synthesegasstrom 13, dessen Hauptteil 18 Kompressorstufe 41 zur Verdichtung auf Synthesedruck zugeführt wird und von dem ein Teil als wasserstoffreicher Synthesegasteilstrom 14 abgezweigt und mit dem Spülgasstrom 15 zusammengeführt wird. Aus den Strömen 14 und 15 resultiert der gemischte Synthesegasstrom 16. Der wasserstoffreiche Synthesegasstrom 18 sowie der Restgasstrom werden gemeinsam einer Kompressorstufe 41 zugeführt. Kompressorstufe 41 verfügt saugseitig über zwei Stutzen, was die gleichzeitige Verdichtung des wasserstoffreichen Synthesegasstroms 18 sowie des Restgasstroms 28 ermöglicht, wobei der kombinierte Synthesegasstrom 20 erhalten wird, welcher in Wärmeaustauscher 33 aufgeheizt und als Synthesegasstrom 21 dem Methanol-Reaktor 34 zugeführt wird.

Bezüglich der weiteren in Figur 2 dargestellten Elemente gelten die in Zusammenhang mit Figur 1 gemachten Ausführungen.

Figur 3 zeigt eine aus dem Stand der Technik bekannte Art der Verfahrensführung. Hierbei wird der Wasserstoffrückgewinnungsstufe 31 ebenfalls ein gemischter Gasstrom aus Synthesegas und Spülgas zugeführt und zur Wasserstoffrückgewinnung genutzt. Jedoch handelt es sich bei dem Synthesegas-Anteil des gemischten Gasstroms um einen Teilfrischgasstrom 60, welcher aus dem (Haupt-)Frischgasstrom 11 mit Hilfe des Drosselorgans 70 abgezweigt

wird. Der Teilfrischgasstrom 60 und der Spülgasstrom 15 werden zusammengeführt und als gemischter Synthesegasstrom 61 der Wasserstoffrückgewinnungsstufe 31 zugeführt. Im Gegensatz zu oben dargestellten erfindungsgemäßen Beispielen wird der gemischte Synthesegasstrom 61 demnach nicht aus bereits mit Wasserstoff angereichertem Synthesegas und Spülgas erzeugt, sondern aus Frischgas und Spülgas. Wie in den nachfolgenden Zahlenbeispielen dargestellt, weist diese Art der Verfahrensführung jedoch Nachteile gegenüber dem erfindungsgemäßen Verfahren auf. Ein Nachteil ergibt sich aus der zwangsläufigen Verwendung des für die Drosselung des (Haupt-)Frischgasstroms 11 erforderlichen Drosselorgans 70. Die Minderung des Drucks durch Drosselorgan 70 muss durch Kompressorstufe 30 kompensiert werden.

Die Vorteile der Erfindung werden im Folgenden durch zwei Zahlenbeispiele aufgezeigt. Beide Beispiele stellen simulierte Fälle da, welche mit Hilfe der Simulationssoftware "Aspen Plus" berechnet wurden.

Beispiel 1

Beispiel 1 basiert auf der Verfahrensführung gemäß Figur 1, im Vergleich mit der Verfahrensführung gemäß Stand der Technik (Figur 3 - Vergleichsbeispiel).

Der wasserstoffarme Synthesegasstrom oder Frischgasstrom (11) weist gemäß Beispiel 1 und Vergleichsbeispiel folgende Zusammensetzung auf:

| Komponente | Anteil (Vol.-%) |
|---|---|
| Wasser | 0,21 |
| Kohlendioxid | 8,04 |
| Kohlenmonoxid | 23,16 |
| Wasserstoff | 65,94 |
| Argon | 0,12 |
| Stickstoff | 0,52 |
| Methan | 2,01 |

Daraus ergibt sich für den wasserstoffarmen Synthesegasstrom oder Frischgasstrom eine Stöchiometriezahl SN von 1,86.

Argon, Stickstoff sowie Methan stellen unter den Bedingungen der Methanolsynthese inerte Gasbestandteile dar und werden im Wesentlichen über den Spülgasstrom (15) aus dem Synthesekreislauf abgeführt.

Der wasserstoffreiche Synthesegasstrom (13, 18) weist gemäß Beispiel 1 und Vergleichsbeispiel folgende Zusammensetzung auf:

| Komponente | Anteil (Vol.-%) |
|---|---|
| Wasser | 0,19 |
| Kohlendioxid | 7,54 |
| Kohlenmonoxid | 21,71 |
| Wasserstoff | 68,07 |
| Argon | 0,11 |
| Stickstoff | 0,49 |
| Methan | 1,89 |

Daraus ergibt sich für den wasserstoffreichen Synthesegasstrom eine Stöchiometriezahl SN von 2,07.

Der Wasserstoffrückgewinnungsstufe (31) zugeführte Stoffmengenstrom an wasserstoffreichem Synthesegas (Anteil 14 am Gesamtstrom des wasserstoffreichen Synthesegases) beträgt 1451,5 kmol/h. Der Stoffmengenstrom des Spülgasstroms (15) beträgt 1306,3 kmol/h. Beide Ströme zusammen bilden den gemischten Synthesegasstrom (16) mit einem Stoffmengenstrom von 2757,8 kmol/h. Daraus ergibt sich für Beispiel 1 ein Stoffmengenstrom-Anteil oder Stoffmengen-Anteil des wasserstoffreichen Synthesegasstroms am gemischten Synthesegasstrom von 0,53.

Der Stoffmengenstrom-Anteil oder Stoffmengen-Anteil des vom wasserstoffreichen Synthesegasstrom abgetrennten Teil (14) in Bezug auf den gesamten Stoffmengenstrom an wasserstoffreichem Synthesegas (13) beträgt gemäß Beispiel 1 0,059. Bezüglich des Energieverbrauchs ergibt sich im Vergleich zur Verfahrensführung gemäß Vergleichsbeispiel (Figur 3) bei gleicher Produktionsmenge an Rohmethanol (Rohmethanol = Gemisch aus Methanol und Wasser) folgendes Bild:

| **Parameter** | **Vergleichsbeispiel (Figur 3)** | Beispiel 1 (Figur 1) |
|---|---|---|
| Kompressorleistung Synthesegas / KW | 16808 | 15791 |
| Massenstrom Erdgas für Frischgaserzeugung/ kg/h | 126801 | 126801 |
| Hochdruckdampfexport-Potential / kg/h | 247190 | 249861 |
| Rohmethanol Produktion / kg/h | 209266 | 209266 |
| Spezifische Kompressorleistung für Kompressorstufe 30 / kW/MT (MT = metrische Tonne) | 80,32 | 75,46 |

Auf Basis der erzielten Energieeinsparungen bezüglich der für die Verdichtung des Synthesegases auf Synthesedruck erforderlichen Kompressorleistung (Kompressorstufe 30 in Figur 1 und Figur 3) ergibt sich eine jährliche Energieeinsparung von 71867 GJ (71867 GJ/a). Zusätzlich bietet die Verfahrensführung gemäß Beispiel 1 (Figur 1) ein höheres Potential zur Erzeugung von Hochdruckdampf als Exportdampf.

Beispiel 2

Beispiel 2 basiert auf der Verfahrensführung gemäß Figur 2, im Vergleich mit der Verfahrensführung gemäß Stand der Technik (Figur 3 - Vergleichsbeispiel).

Der wasserstoffarme Synthesegasstrom oder Frischgasstrom (11) weist gemäß Beispiel 2 und Vergleichsbeispiel folgende Zusammensetzung auf:

| **Komponente** | **Anteil (Vol.-%)** |
|---|---|
| Wasser | 0,16 |
| Kohlendioxid | 7,54 |
| Kohlenmonoxid | 24,68 |
| Wasserstoff | 65,55 |
| Argon | 0,12 |
| Stickstoff | 0,09 |
| Methan | 1,86 |

Daraus ergibt sich für den wasserstoffarmen Synthesegasstrom oder Frischgasstrom eine Stöchiometriezahl SN von 1,80.

Der wasserstoffreiche Synthesegasstrom (13, 18) weist gemäß Beispiel 2 und Vergleichsbeispiel folgende Zusammensetzung auf:

| **Komponente** | **Anteil (Vol.-%)** |
|---|---|
| Wasser | 0,14 |
| Kohlendioxid | 6,99 |
| Kohlenmonoxid | 22,84 |
| Wasserstoff | 68,12 |
| Argon | 0,11 |
| Stickstoff | 0,08 |

(fortgesetzt)

| Komponente | Anteil (Vol.-%) |
|---|---|
| Methan | 1,72 |

Daraus ergibt sich für den wasserstoffreichen Synthesegasstrom eine Stöchiometriezahl SN von 2,05.

Der Wasserstoffrückgewinnungsstufe (31) zugeführte Stoffmengenstrom an wasserstoffreichem Synthesegas (Anteil 14 am Gesamtstrom des wasserstoffreichen Synthesegases) beträgt 2280,0 kmol/hr. Der Stoffmengenstrom des Spülgasstroms (15) beträgt 976,4 kmol/hr. Beide Ströme zusammen bilden den gemischten Synthesegasstrom (16) mit einem Stoffmengenstrom von 3256,4 kmol/hr. Daraus ergibt sich für Beispiel 2 ein Stoffmengenstrom-Anteil oder Stoffmengen-Anteil des wasserstoffreichen Synthesegasstroms am gemischten Synthesegasstrom von 0,70.

Der Stoffmengenstrom-Anteil oder Stoffmengen-Anteil des vom wasserstoffreichen Synthesegasstrom abgetrennten Teil (14) in Bezug auf den gesamten Stoffmengenstrom an wasserstoffreichem Synthesegas (13) beträgt gemäß Beispiel 2 0,091.

Bezüglich des Energieverbrauchs ergibt sich im Vergleich zur Verfahrensführung gemäß Vergleichsbeispiel (Figur 3) bei gleicher Produktionsmenge an Rohmethanol (Rohmethanol = Gemisch aus Methanol und Wasser) folgendes Bild:

| Parameter | Vergleichsbeispiel (Figur 3) | Beispiel 2 (Figur 2) |
|---|---|---|
| Kompressorleistung Synthesegas / KW | 10756 | 8797 |
| Leistung Wasserstoffkompressor | n/a | 84 |
| Massenstrom Erdgas für Frischgaserzeugung/ kg/h | 131664 | 131147 |
| Rohmethanol Produktion / kg/h | 209394 | 209394 |
| Spezifische Kompressorleistung für Kompressorstufe 30 / kW/MT (MT = metrische Tonne) | 51,37 | 42,42 |

Auf Basis der erzielten Energieeinsparungen bezüglich der für die Verdichtung des Synthesegases auf Synthesedruck erforderlichen Kompressorleistung (Kompressorstufe 30 in Figur 3; Synthesegas-Anteil Kompressorstufe 41 sowie Wasserstoffkompressor 40 in Figur 2) ergibt sich eine jährliche Energieeinsparung von 206548 GJ (206548 GJ/a).

**Bezugszeichenliste**

| | |
|---|---|
| 100, 200 | Verfahren, Anlage (Erfindung) |
| 300 | Verfahren, Anlage (Stand der Technik) |
| 11 | Frischgasstrom |
| 12, 51 | wasserstoffhaltiger Strom |
| 13, 18 | wasserstoffreicher Synthesegasstrom |
| 14 | wasserstoffreicher Synthesegasteilstrom |
| 15 | Spülgasstrom |
| 16 | gemischter Synthesegasstrom (Erfindung) |
| 17 | Abgas |
| 19, 26, 28 | Restgasstrom |
| 20 | Kombinierter Synthesegasstrom |
| 21 | Synthesegasstrom |
| 22, 23, 24, 25 | Produktstrom |
| 26 | Restgasstrom |
| 27 | Rohmethanolstrom |
| 30, 32, 41 | Kompressorstufe |
| 31 | Wasserstoffrückgewinnungsstufe |

| | |
|---|---|
| 33, 36, 37 | Wärmeausstauscher |
| 38 | Abscheider |
| 40 | Wasserstoffkompressor |
| 60 | Teilfrischgasstrom |
| 61 | gemischter Synthesegasstrom (Stand der Technik) |
| 70 | Drosselorgan |

## Patentansprüche

1. Verfahren (100, 200) zur Herstellung von Methanol, wobei einem Frischgasstrom (11) aus einer Reformereinheit, welcher Wasserstoff und Kohlenoxide umfasst, ein wasserstoffhaltiger Strom (12, 51) aus einer Wasserstoffrückgewinnungsstufe (31) zugeführt wird, wobei ein wasserstoffreicher Synthesegasstrom (13, 18) mit einer Stöchiometriezahl SN, definiert als SN = $[n(H_2) - n(CO_2)] / [n(CO) + n(CO_2)]$, von 2.0 oder größer erhalten wird, und wobei der wasserstoffreiche Synthesegasstrom mit einem Restgasstrom (19, 28) zusammengeführt und der wasserstoffreiche Synthesegasstrom und der Restgasstrom bei erhöhtem Druck und erhöhter Temperatur durch ein Bett eines Methanolsynthese-Katalysators (35) geleitet werden, wobei ein Produktstrom (22, 23, 24, 25) erhalten wird, welcher Methanol und den Restgasstrom umfasst, und wobei der Produktstrom gekühlt wird um Methanol (27) vom Restgasstrom abzutrennen,
**dadurch gekennzeichnet, dass**
von dem Restgasstrom ein Teil als Spülgasstrom (15) abgetrennt wird und von dem wasserstoffreichen Synthesegasstrom ein Teil (14) abgetrennt und mit dem Spülgasstrom zusammengeführt wird, wobei ein gemischter Synthesegasstrom (16) erhalten wird, und der gemischte Synthesegasstrom der Wasserstoffrückgewinnungsstufe zur Erzeugung des wasserstoffhaltigen Stroms zugeführt wird.

2. Verfahren nach Anspruch 1, **dadurch gekennzeichnet, dass** der wasserstoffreiche Synthesegasstrom verdichtet wird, und von dem verdichteten wasserstoffreichen Synthesegasstrom (18) ein Teil abgetrennt und mit dem Spülgasstrom zusammengeführt wird.

3. Verfahren nach Anspruch 2, **dadurch gekennzeichnet, dass** der Restgasstrom verdichtet und mit dem verdichteten wasserstoffreichen Synthesegasstrom zusammengeführt wird und die zusammengeführten Ströme durch das Bett des Methanolsynthese-Katalysators geleitet werden.

4. Verfahren nach Anspruch 1, **dadurch gekennzeichnet, dass** der wasserstoffhaltige Strom durch einen Wasserstoffkompressor (40) verdichtet wird, und der verdichtete wasserstoffhaltige Strom mit dem Frischgasstrom zusammengeführt wird, wobei der wasserstoffreiche Synthesegasstrom erhalten wird, und von dem wasserstoffreichen Synthesegasstrom ein Teil abgetrennt und mit dem Spülgasstrom zusammengeführt wird.

5. Verfahren nach Anspruch 4, **dadurch gekennzeichnet, dass** der wasserstoffreiche Synthesegasstrom und der Restgasstrom gemeinsam verdichtet und durch das Bett des Methanolsynthese-Katalysators geleitet werden.

6. Verfahren nach einem der vorhergehenden Ansprüche, **dadurch gekennzeichnet, dass** der Stoffmengenstrom-Anteil des wasserstoffreichen Synthesegasstroms am gemischten Synthesegasstrom zwischen 0,10 und 0,95 liegt, bevorzugt zwischen 0,20 und 0,90 liegt, weiter bevorzugt zwischen 0,30 und 0,80 liegt und weiter bevorzugt zwischen 0,50 und 0,75 liegt.

7. Verfahren nach einem der vorhergehenden Ansprüche, **dadurch gekennzeichnet, dass** der Stoffmengenstrom-Anteil des vom wasserstoffreichen Synthesegasstrom abgetrennten Teils in Bezug auf den gesamten Stoffmengenstrom an wasserstoffreichem Synthesegas zwischen 0,001 und 0,999 liegt, vorzugsweise zwischen 0,005 und 0,800 liegt, weiter bevorzugt zwischen 0,010 und 0,500 liegt, weiter bevorzugt zwischen 0,020 und 0,200 liegt und weiter bevorzugt zwischen 0,050 und 0,100 liegt.

8. Verfahren nach einem der vorhergehenden Ansprüche, **dadurch gekennzeichnet, dass** der wasserstoffreiche Synthesegasstrom eine Stöchiometriezahl SN von 2,00 bis 2,20 aufweist, bevorzugt von 2,02 bis 2,10 aufweist, und weiter bevorzugt von 2,05 bis 2,07 aufweist.

9. Verfahren nach einem der vorhergehenden Ansprüche, **dadurch gekennzeichnet, dass** der Frischgasstrom eine Stöchiometriezahl SN von kleiner als 2,0 aufweist, bevorzugt von 1,70 bis 1,95 aufweist, weiter bevorzugt von 1,75

bis 1,90 aufweist, und weiter bevorzugt von 1,78 bis 1,85 aufweist.

**10.** Verfahren nach einem der vorhergehenden Ansprüche, **dadurch gekennzeichnet, dass** die Wasserstoffrückgewinnungsstufe eine Wechseldruckabsorptionsvorrichtung zur Abtrennung von Wasserstoff aus dem gemischten Synthesegasstrom umfasst.

**11.** Verfahren nach einem der Ansprüche 1 bis 9, **dadurch gekennzeichnet, dass** die Wasserstoffrückgewinnungsstufe eine Membrantrennstufe zur Abtrennung von Wasserstoff aus dem gemischten Synthesegasstrom umfasst.

**12.** Verfahren nach einem der vorhergehenden Ansprüche, **dadurch gekennzeichnet, dass** der wasserstoffhaltige Strom einen Wasserstoffanteil von mindestens 95 Vol.-% aufweist, bevorzugt von mindestens 99 Vol.-% aufweist, weiter bevorzugt von mindestens 99,5 Vol.-% aufweist, weiter bevorzugt von mindestens 99,9 Vol.-% aufweist.

**13.** Eine Anlage (100, 200) zur Herstellung von Methanol, aufweisend folgende miteinander in Fluidverbindung stehende Anlagenkomponenten:

Eine Reformereinheit zur Erzeugung eines Wasserstoff und Kohlenstoffoxide aufweisenden Frischgasstroms (11);
eine Wasserstoffrückgewinnungsstufe (31) zur Erzeugung eines wasserstoffhaltigen Stroms (12, 51), wobei die Reformereinheit und die Wasserstoffrückgewinnungsstufe so konfiguriert sind, dass aus dem wasserstoffhaltigen Strom und dem Frischgasstrom ein wasserstoffreicher Synthesegasstrom (13) mit einer Stöchiometriezahl SN, definiert als SN = $[n(H_2) - n(CO_2)] / [n(CO) + n(CO_2)]$, von 2.0 oder größer erhältlich ist;
eine Reaktorstufe (34) umfassend ein Methanolsynthese-Katalysatorbett (35), wobei die Reaktorstufe so konfiguriert ist, dass der wasserstoffreiche Synthesegasstrom sowie ein Restgasstrom (19, 26, 28) bei erhöhtem Druck und erhöhter Temperatur durch das Methanolsynthese-Katalysatorbett hindurchgeleitet werden können, wodurch
ein Produktstrom (22, 23, 24, 25) erhältlich ist, welcher Methanol und den Restgasstrom umfasst;
eine Kühlvorrichtung (33, 36, 37) zum Kühlen des Produktstroms, wobei die Kühlvorrichtung so konfiguriert ist, dass Methanol (27) vom Restgasstrom abgetrennt werden kann,
**dadurch gekennzeichnet, dass**
die Anlage so konfiguriert ist, dass von dem Restgasstrom ein Teil als Spülgasstrom abgetrennt werden kann und von dem Synthesegasstrom ein Teil abgetrennt und mit dem Spülgasstrom zusammengeführt werden kann, wodurch ein gemischter Synthesegasstrom (16) erhältlich ist, und der gemischte Synthesegasstrom der Wasserstoffrückgewinnungsstufe zur Erzeugung des wasserstoffhaltigen Stroms zugeführt werden kann.

**14.** Verwendung des Verfahrens nach einem der Ansprüche 1 bis 12 oder der Anlage nach Anspruch 13 zur Herstellung von Methanol aus durch autotherme Reformierung und/oder partielle Oxidation erzeugtem Frischgas.

## Claims

**1.** Process (100, 200) for producing methanol, wherein a make-up gas stream (11) from a reformer unit comprising hydrogen and carbon oxides is admixed with a hydrogen-containing stream (12, 51) from a hydrogen recovery stage (31) to obtain a hydrogen-rich synthesis gas stream (13, 18) having a stoichiometry number SN, defined as SN = $[n(H_2) - n(CO_2)] / [n(CO) + n(CO_2)]$, of not less than 2.0 and wherein the hydrogen-rich synthesis gas stream is combined with a residual gas stream (19, 28) and the hydrogen-rich synthesis gas stream and the residual gas stream are passed through a bed of a methanol synthesis catalyst (35) at elevated pressure and elevated temperature to obtain a product stream (22, 23, 24, 25) comprising methanol and the residual gas stream and wherein the product stream is cooled to remove methanol (27) from the residual gas stream, **characterized in that**

a portion of the residual gas stream is removed as a purge gas stream (15) and
a portion (14) of the hydrogen-rich synthesis gas stream is removed and combined with the purge gas stream to obtain a mixed synthesis gas stream (16) and the mixed synthesis gas stream is sent to the hydrogen recovery stage to produce the hydrogen-containing stream.

**2.** Process according to Claim 1, **characterized in that** the hydrogen-rich synthesis gas stream is compressed and a portion of the compressed hydrogen-rich synthesis gas stream (18) is removed and combined with the purge gas stream.

3. Process according to Claim 2, **characterized in that** the residual gas stream is compressed and combined with the compressed hydrogen-rich synthesis gas stream and the combined streams are passed through the bed of the methanol synthesis catalyst.

4. Process according to Claim 1, **characterized in that** the hydrogen-containing stream is compressed by a hydrogen compressor (40) and the compressed hydrogen-containing stream is combined with the make-up gas stream to obtain the hydrogen-rich synthesis gas stream and a portion of the hydrogen-rich synthesis gas stream is removed and combined with the purge gas stream.

5. Process according to Claim 4, **characterized in that** the hydrogen-rich synthesis gas stream and the residual gas stream are compressed and passed through the bed of the methanol synthesis catalyst together.

6. Process according to any of the preceding claims, **characterized in that** the molar flow rate proportion of the hydrogen-rich synthesis gas stream in the mixed synthesis gas stream is between 0.10 and 0.95, preferably between 0.20 and 0.90, more preferably between 0.30 and 0.80 and more preferably between 0.50 and 0.75.

7. Process according to any of the preceding claims, **characterized in that** the molar flow rate proportion of the portion removed from the hydrogen-rich synthesis gas stream based on the total molar flow rate of hydrogen-rich synthesis gas is between 0.001 and 0.999, preferably between 0.005 and 0.800, more preferably between 0.010 and 0.500, more preferably between 0.020 and 0.200 and more preferably between 0.050 and 0.100.

8. Process according to any of the preceding claims, **characterized in that** the hydrogen-rich synthesis gas stream has a stoichiometry number SN of 2.00 to 2.20, preferably of 2.02 to 2.10 and more preferably of 2.05 to 2.07.

9. Process according to any of the preceding claims, **characterized in that** the make-up gas stream has a stoichiometry number SN of less than 2.0, preferably of 1.70 to 1.95, more preferably of 1.75 to 1.90 and more preferably of 1.78 to 1.85.

10. Process according to any of the preceding claims, **characterized in that** the hydrogen recovery stage comprises a pressure swing absorption apparatus for removing hydrogen from the mixed synthesis gas stream.

11. Process according to any of Claims 1 to 9, **characterized in that** the hydrogen recovery stage comprises a membrane separation stage for removing hydrogen from the mixed synthesis gas stream.

12. Process according to any of the preceding claims, **characterized in that** the hydrogen-containing stream has a hydrogen proportion of at least 95% by volume, preferably of at least 99% by volume, more preferably of at least 99.5% by volume, more preferably of at least 99.9% by volume.

13. Plant (100, 200) for producing methanol comprising the following plant components arranged in fluid connection with one another:

a reformer unit for producing a make-up gas stream (11) comprising hydrogen and carbon oxides;
a hydrogen recovery stage (31) for producing a hydrogen-containing stream (12, 51), wherein the reformer unit and the hydrogen recovery stage are configured such that a hydrogen-rich synthesis gas stream (13) having a stoichiometry number SN, defined as $SN = [n(H_2) - n(CO_2)] / [n(CO) + n(CO_2)]$, of not less than 2.0 is obtainable from the hydrogen-containing stream and the make-up gas stream;
a reactor stage (34) comprising a methanol synthesis catalyst bed (35), wherein the reactor stage is configured such that the hydrogen-rich synthesis gas stream and a residual gas stream (19, 26, 28) may be passed through the methanol synthesis catalyst bed at elevated pressure and elevated temperature, thus making it possible to obtain a product stream (22, 23, 24, 25) comprising methanol and the residual gas stream;
a cooling apparatus (33, 36, 37) for cooling the product stream, wherein the cooling apparatus is configured such that methanol (27) may be removed from the residual gas stream,
**characterized in that**
the plant is configured such that a portion of the residual gas stream may be removed as a purge gas stream and a portion of the synthesis gas stream may be removed and combined with the purge gas stream, thus making it possible to obtain a mixed synthesis gas stream (16), and the mixed synthesis gas stream may be sent to the hydrogen recovery stage to produce the hydrogen-containing stream.

14. Use of the process according to any of Claims 1 to 12 or of the plant according to Claim 13 for producing methanol from make-up gas produced by autothermal reforming and/or partial oxidation.

## Revendications

1. Procédé (100, 200) pour la production de méthanol, un courant contenant de l'hydrogène (12, 51) provenant d'une étape de récupération d'hydrogène (31) étant ajouté à un courant de gaz frais (11) provenant d'une unité de reformage, qui comprend de l'hydrogène et des oxydes de carbone, un courant de gaz de synthèse riche en hydrogène (13, 18) ayant un nombre stœchiométrique SN, défini par SN = $[n(H_2) - n(CO_2)]/[n\ (CO) + n(CO_2)]$, de 2,0 ou plus étant obtenu, et le courant de gaz de synthèse riche en hydrogène étant combiné à un courant de gaz résiduel (19, 28) et le courant de gaz de synthèse riche en hydrogène et le courant de gaz résiduel étant passés à pression élevée et température élevée à travers un lit d'un catalyseur de synthèse de méthanol (35), un courant de produit (22, 23, 24, 25) étant obtenu, qui comprend du méthanol et le courant de gaz résiduel, et le courant de produit étant refroidi pour séparer le méthanol (27) du courant de gaz résiduel,
**caractérisé en ce que**
une partie du courant de gaz résiduel est séparée en tant que courant de gaz de balayage (15) et une partie (14) du courant de gaz de synthèse riche en hydrogène est séparée et combinée avec le courant de gaz de balayage, un courant de gaz de synthèse mélangé (16) étant obtenu, et le courant de gaz de synthèse mélangé est amené à l'étape de récupération d'hydrogène pour produire le courant contenant de l'hydrogène.

2. Procédé selon la revendication 1, **caractérisé en ce que** le courant de gaz de synthèse riche en hydrogène est comprimé, et une partie du courant de gaz de synthèse riche en hydrogène comprimé (18) est séparée et combinée avec le courant de gaz de balayage.

3. Procédé selon la revendication 2, **caractérisé en ce que** le courant de gaz résiduel est comprimé et combiné avec le courant de gaz de synthèse riche en hydrogène comprimé, et les courants combinés sont passés à travers le lit du catalyseur de synthèse de méthanol.

4. Procédé selon la revendication 1, **caractérisé en ce que** le courant contenant de l'hydrogène est comprimé par un compresseur d'hydrogène (40), et le courant contenant de l'hydrogène comprimé est combiné avec le courant de gaz frais, le courant de gaz de synthèse riche en hydrogène étant obtenu, et une partie du courant de gaz de synthèse riche en hydrogène est séparée et combinée avec le courant de gaz de balayage.

5. Procédé selon la revendication 4, **caractérisé en ce que** le courant de gaz de synthèse riche en hydrogène et le courant de gaz résiduel sont comprimés ensemble et passés à travers le lit du catalyseur de synthèse de méthanol.

6. Procédé selon l'une quelconque des revendications précédentes, **caractérisé en ce que** la proportion de courant de matière du courant de gaz de synthèse riche en hydrogène par rapport au courant de gaz de synthèse mélangé est comprise entre 0,10 et 0,95, de préférence entre 0,20 et 0,90, de manière davantage préférée entre 0,30 et 0,80 et de manière davantage préférée entre 0,50 et 0,75.

7. Procédé selon l'une quelconque des revendications précédentes, **caractérisé en ce que** la proportion de courant de matière de la partie séparée du courant de gaz de synthèse riche en hydrogène par rapport au courant de matière total de gaz de synthèse riche en hydrogène est comprise entre 0,001 et 0,999, de préférence entre 0,005 et 0,800, de manière davantage préférée entre 0,010 et 0,500, de manière davantage préférée entre 0,020 et 0,200 et de manière davantage préférée entre 0,050 et 0,100.

8. Procédé selon l'une quelconque des revendications précédentes, **caractérisé en ce que** le courant de gaz de synthèse riche en hydrogène présente un nombre stœchiométrique SN de 2,00 à 2,20, de préférence de 2,02 à 2,10, et de manière davantage préférée de 2,05 à 2,07.

9. Procédé selon l'une quelconque des revendications précédentes, **caractérisé en ce que** le courant de gaz frais présente un nombre stœchiométrique SN inférieur à 2,0, de préférence de 1,70 à 1,95, de manière davantage préférée de 1,75 à 1,90, et de manière davantage préférée de 1,78 à 1,85.

10. Procédé selon l'une quelconque des revendications précédentes, **caractérisé en ce que** l'étape de récupération d'hydrogène comprend un dispositif d'absorption à pression alternée pour séparer l'hydrogène du courant de gaz

de synthèse mélangé.

**11.** Procédé selon l'une quelconque des revendications 1 à 9, **caractérisé en ce que** l'étape de récupération d'hydrogène comprend une étape de séparation à membrane pour séparer l'hydrogène du courant de gaz de synthèse mélangé.

**12.** Procédé selon l'une quelconque des revendications précédentes, **caractérisé en ce que** le courant contenant de l'hydrogène présente une proportion d'hydrogène d'au moins 95 % en volume, de préférence d'au moins 99 % en volume, de manière davantage préférée d'au moins 99,5 % en volume, de manière davantage préférée d'au moins 99,9 % en volume.

**13.** Installation (100, 200) pour la production de méthanol, présentant les composants d'installation suivants en liaison fluidique les uns avec les autres :

une unité de reformage pour produire un courant de gaz frais (11) comprenant de l'hydrogène et des oxydes de carbone ;
une étape de récupération d'hydrogène (31) pour produire un courant contenant de l'hydrogène (12, 51), l'unité de reformage et l'étape de récupération d'hydrogène étant configurées de telle sorte qu'à partir du courant contenant de l'hydrogène et du courant de gaz frais, un courant de gaz de synthèse riche en hydrogène (13) ayant un nombre stœchiométrique SN, défini par SN = $[n(H_2) - n(CO_2)]/[n(CO) + n(CO_2)]$, de 2,0 ou plus peut être obtenu ;
une étape de réacteur (34) comprenant un lit de catalyseur de synthèse de méthanol (35), l'étape de réacteur étant configurée de telle sorte que le courant de gaz de synthèse riche en hydrogène et un courant de gaz résiduel (19, 26, 28) peuvent être passés à pression élevée et température élevées à travers le lit de catalyseur de synthèse de méthanol, ce qui permet d'obtenir un courant de produit (22, 23, 24, 25), qui comprend du méthanol et le courant de gaz résiduel ;
un dispositif de refroidissement (33, 36, 37) pour refroidir le courant de produit, le dispositif de refroidissement étant configuré de telle sorte que le méthanol (27) peut être séparé du courant de gaz résiduel,
**caractérisée en ce que**
l'installation est configurée de telle sorte qu'une partie du courant de gaz résiduel peut être séparée en tant que courant de gaz de balayage et une partie du courant de gaz de synthèse peut être séparée et combinée avec le courant de gaz de balayage, ce qui permet d'obtenir un courant de gaz de synthèse mélangé (16), et le courant de gaz de synthèse mélangé peut être amené à l'étape de récupération d'hydrogène pour produire le courant contenant de l'hydrogène.

**14.** Utilisation du procédé selon l'une quelconque des revendications 1 à 12 ou de l'installation selon la revendication 13 pour la production de méthanol à partir de gaz frais produit par reformage autotherme et/ou oxydation partielle.

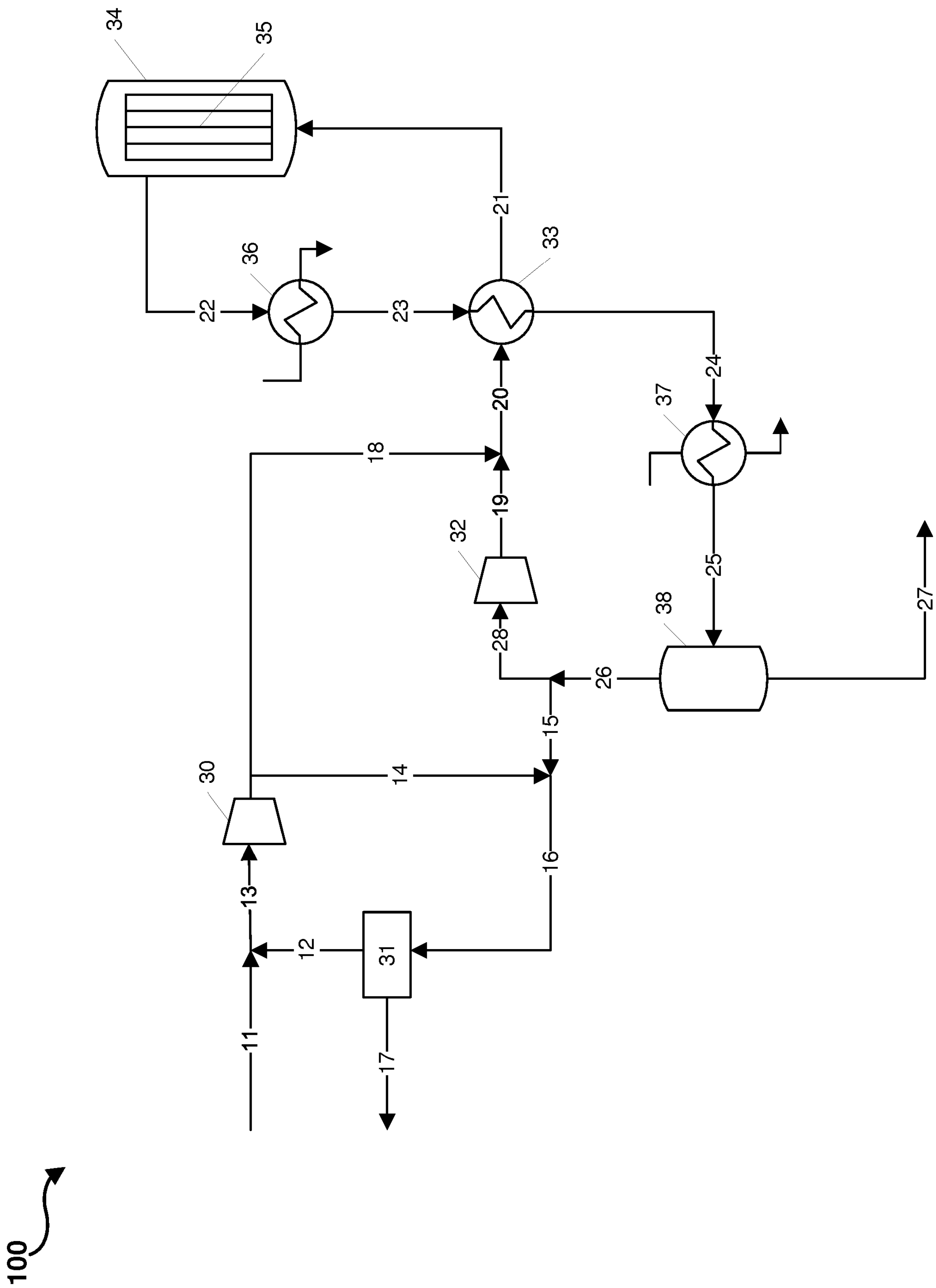
100
11
12
13
14
15
16
17
18
19
20
21
22
23
24
25
26
27
28
30
31
32
33
34
35
36
37
38

Fig. 1

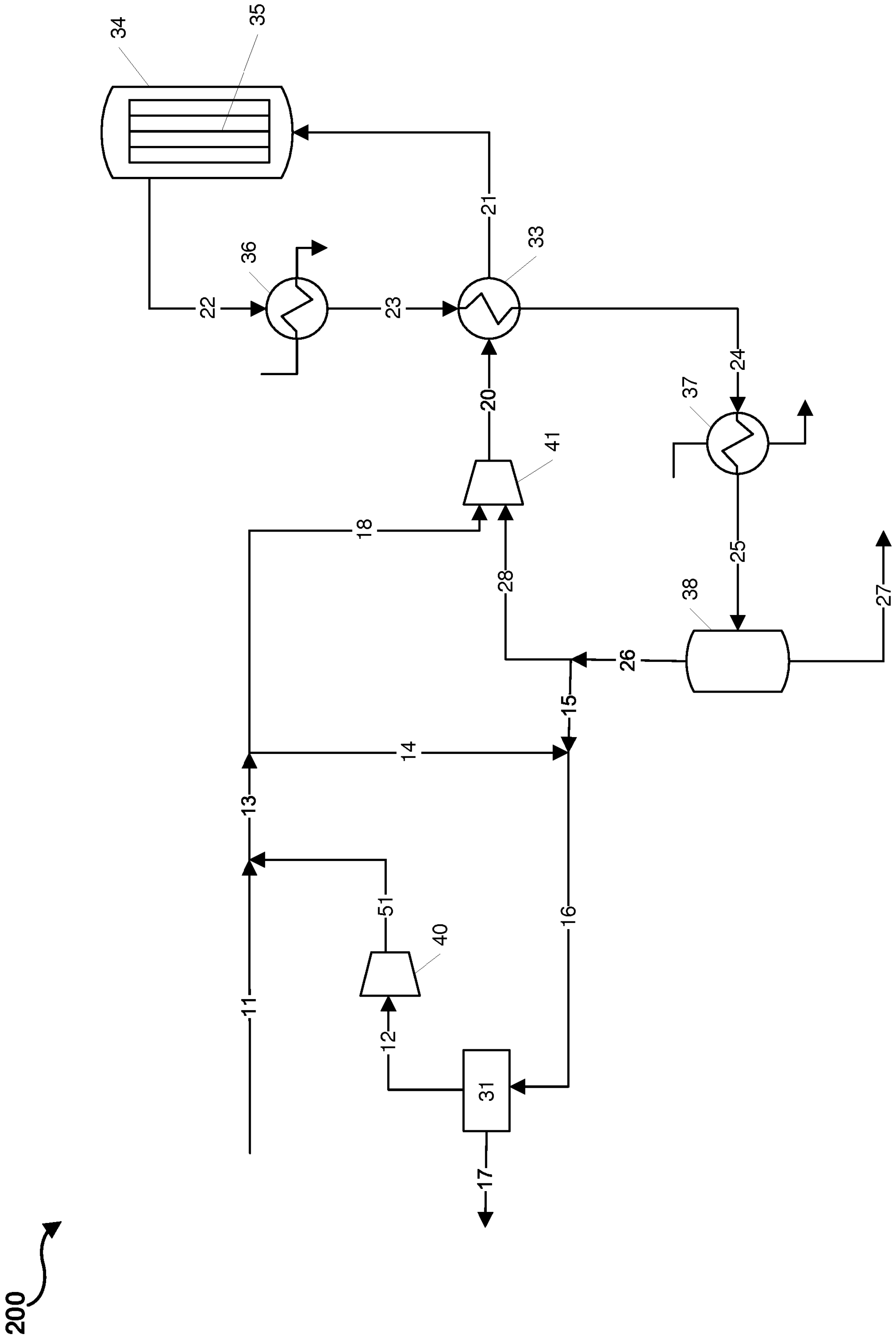
200
11
12
13
14
15
16
17
18
20
21
22
23
24
25
26
27
28
31
33
34
35
36
37
38
40
41
51

Fig. 2

Fig. 3

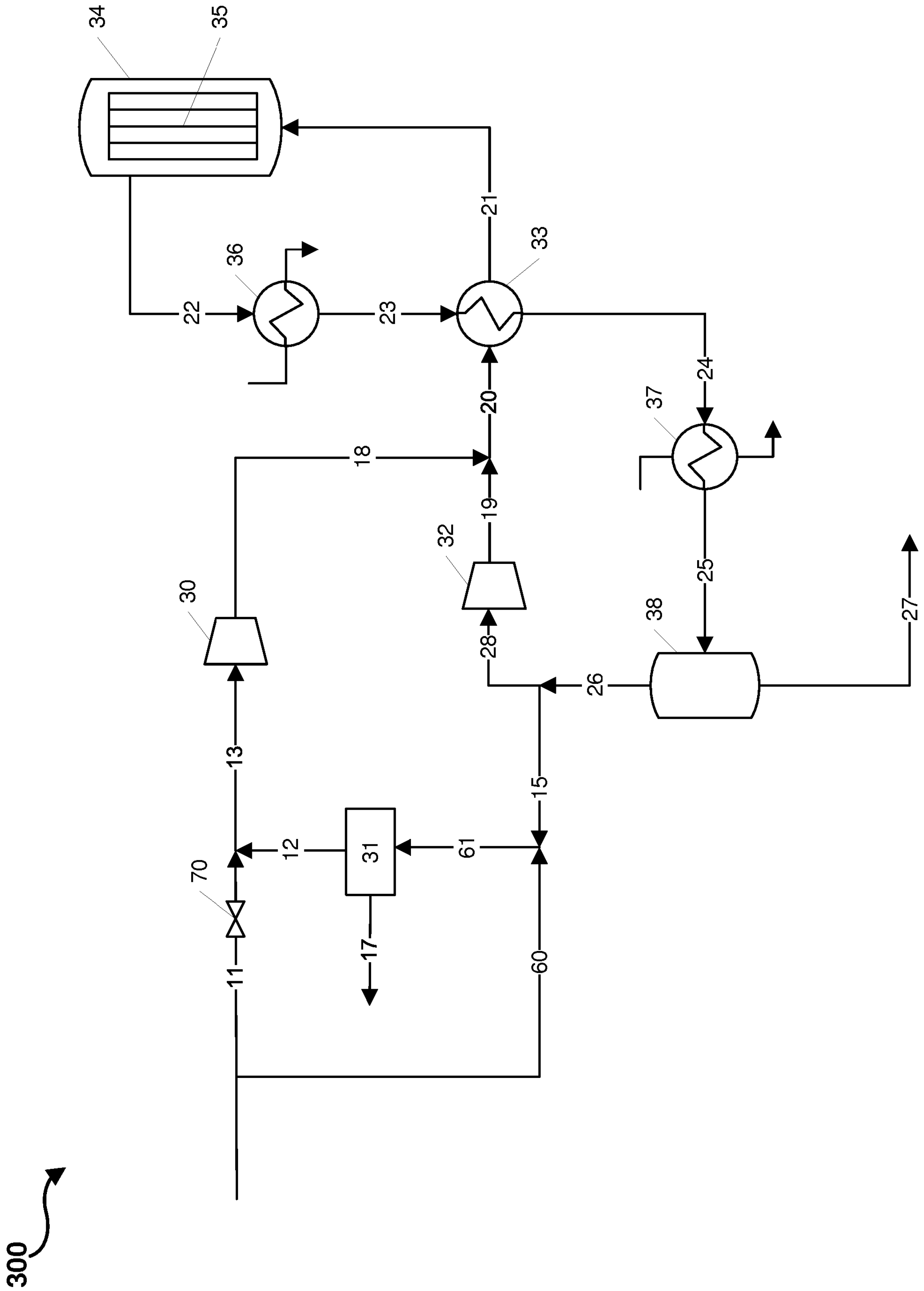
300
11
12
13
15
17
18
19
20
21
22
23
24
25
26
27
28
30
31
32
33
34
35
36
37
38
60
61
70

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 112006001310 T5
- EP 3205622 B1
- US 7786180 B2